# EUROPEAN PATENT SPECIFICATION

(11) **EP 0 979 120 B1**
(45) Date of publication and mention of the grant of the patent: **04.05.2005**
(21) Application number: 98910446.8
(22) Date of filing: 17.03.1998
(51) Int. Cl.: A61M 16/04

(54) **ENDOTRACHEAL TUBE**
ENDOTRACHEALTUBUS
TUBE ENDOTRACHEAL

(30) Priority: 18.03.1997 US 819783
(43) Date of publication of application: 16.02.2000
(73) Proprietor: Parker Medical Limited Partnership, Cincinnati, OH 45208 (US)
(72) Inventor: PARKER, Jeffrey, D., Cincinnati, OH 45208 (US)
(74) Representative: Findlay, Alice Rosemary
(86) International application number: PCT/US1998/005173
(87) International publication number: WO 1998/041273

(56) References cited:
- US-A- 2 458 305
- US-A- 3 964 488
- US-A- 4 231 365
- US-A- 4 423 725
- US-A- 5 044 369
- US-A- 5 414 075
- US-A- 5 720 275

## Description

### Background of the Invention

### 1. Field of the Invention

The present invention relates to endotracheal tubes for ventilation of the lungs, and more particularly, to such tubes suitable for blind guided intubation.

### II. Description of Prior Art

When a patient stops breathing, it is imperative that effective ventilation be instituted as soon as possible. Ventilation is best accomplished by forcing air through an endotracheal tube inserted through the mouth and laryngeal opening and into the trachea (in which case the tube may be referred to as an orotracheal tube). The endotracheal tube is usually a preformed, semi-flexible tubular member having a gas flow lumen extending therethrough. The tube has an arcuate shape imparted to it and the distal tip is usually cut completely across at an angle to define a beveled edge to facilitate its insertion between the vocal cords.

The usual method of orotracheal intubation relies on a blade laryngoscope by which to visualize the laryngeal opening so as to facilitate insertion of the tube. The endotracheal tube used to intubate with the blade laryngoscope is usually introduced into the laryngeal opening from the right side thereof (i.e., the patient's right side). To facilitate this procedure, the distal tip is beveled on its side relative to the curvature of the tube (i.e., the tube is cut with a left side-facing bevel that extends at an angle down from the left aspect of the tube through the right aspect) such that the terminal tip defines a right-sided chisel point, which obstructs the view of the vocal cords as little as possible as it approaches those cords and provides a suitably narrow contour for insertion therebetween, and a left-sided elliptical hole circumscribed by the left side-facing beveled edge. Intubation with the blade laryngoscope presents significant difficulties and risks, however. In addition to possible injury or trauma to the patient in the utilization of the blade laryngoscope, it is not uncommon for the broad, rigid tip of the orotracheal tube to traumatize the larynx during its attempted advancement therethrough. It is also not uncommon for the orotracheal tube to be accidentally inserted into anatomical spaces surrounding the larynx, such as the closely adjacent esophagus. Such misintubation, if not quickly recognized and corrected, may have fatal consequences.

Another approach to intubation is so-called blind orotracheal intubation in which a guide device is inserted into the throat to guide the orotracheal tube into the laryngeal opening without requiring visualization of the laryngeal opening. Blind intubation guides have been developed which both minimize injury and trauma in use, and also substantially reduce the risk of misintubation. Such intubation guides are shown, for example, in U. S. Patent No. 5,339,805 and U.S. Patent 5 743 254. With some blind intubation guides, the endotracheal tube is advanced through the guide into the laryngeal opening along the midline of that opening rather than obliquely from the side of that opening, as with a blade laryngoscope. Thus, the side-facing bevel of the tube tip may be of no useful benefit and, indeed, may be disadvantageous in that the right-sided chisel point may become impacted on the right side of the larynx, and the left-facing elliptical hole may snag on left-sided laryngeal features, such as the left arytenoid and corniculate cartilages, thus preventing the tube from advancing into the trachea.

A visual means to facilitate intubation is to insert the imaging bundle of a flexible fiberoptic bronchoscope into the lumen of an orotracheal tube, so that the imaging lens of the scope is positioned just inside the distal opening of the tube. This allows the bronchoscopist to see whatever is immediately in front of the tip of the tube. The tube, containing this imaging means, is then inserted into the throat with a blind intubation guide such as the devices in the aforesaid '805 patent and '737 patent application referenced above. The intubation guide conducts the tip to a position directly above the larynx, so that the bronchoscopist can immediately see the vocal cords and advance the scope-containing tube tip there-through into the trachea. The scope is then withdrawn leaving the tube in place. For this technique to work, however, there must be an unobstructed view provided to the bronchoscope by and through the distal opening of the tube. Some endotracheal tubes have been proposed with left and right-sided, opposed bevels and having a transverse strut therebetween such that left and right-sided openings are defined at the distal end of the tube. One such device is shown in U.S. Patent No. 2,862,498. The strut, while necessary for the operation of that endotracheal tube, renders the technique of visualizing the vocal cords impossible.

Such a strut also prevents another blind intubation technique in which the tube is railroaded over another member such as a tubular orotracheal introducer. In such a technique, the introducer is inserted into an orotracheal tube and extends through the tube lumen beyond the distal tip. The forward end of the introducer is inserted through the laryngeal opening into the trachea, and the tube is railroaded down over the introducer into the trachea. For such a technique to work, the introducer must remain aligned with the lumen of the tube which is intended to be railroaded over the introducer. The transverse strut deflects the introducer out a side opening thereby preventing the necessary alignment. Railroading the tube over a misaligned introducer will cause the tube to snag on the larynx, resulting in trauma to the larynx and failed intubation.

Another blind intubation technique is nasotracheal intubation in which an endotracheal tube is passed through the nose into the back of the throat and then down into the larynx and trachea (in which case the tube may be referred to as a nasotracheal tube). The nose contains friable mucosa which is especially susceptible to mechanical trauma. Standard, side-beveled tubes, due to the rigidity of their tips, can disrupt this mucosa and provoke substantial nasal bleeding when they are advanced through the nose. Such rigidity also prevents them from gently turning the sharp comer at the back of the nose in order to travel downward toward the larynx. Additional trauma and bleeding can be caused at that site.

Another problem with tubes used for orotracheal and nasotracheal intubation is that they are unlubricated and can therefore cause frictional trauma to anatomical structures during insertion into the body. Intubationists who wish to avoid such friction must pause to manually lubricate the tube before inserting it into the body. The lubricant is not always applied uniformly, and may also be easily rubbed off, as when the tube is advanced through a narrow nostril. As a consequence, manual lubrication does not assure that frictional trauma will be avoided.

An endotracheal tube proposed in U.S. Patent No. 4,050,466 has a rear-facing bevel (i.e., the tube tip is cut at an angle that extends down from the outer surface of the tube's convex posterior wall through the outer surface of the tube's concave anterior wall), rather than a side-facing bevel. The rear-facing bevel moves the chisel point of the bevel tip from a lateral aspect of the tube to the anterior aspect. The chisel point created by this complete posterior bevel is, however, too broad, and too anteriorly disposed. As a result, the tube may have a tendency to become impacted on the posterior base of the epiglottis or hung up on the vocal cords at the anterior commissure of the glottis where the space between the vocal cords is the narrowest. Even if the tube successfully passes through the glottis, the chisel point would tend to become lodged on or between the cartilaginous rings within the trachea thus preventing further advancement in the trachea. Moreover, the tip of the tube is rigid and can, therefore, do substantial damage to structures in the nose and throat when the usual force is applied to advance the tube.

### Summary of the Invention

The present invention in one aspect provides an endotracheal tube comprising a tubular member extending between a proximal end and a distal end and having a single opening across the distal end, the tubular member having a generally predefined curvature with a first wall aspect and a second, oppositely disposed wall aspect along its length define an airway lumen therebetween for flow of gas between the proximal end and the opening, characterised in that the opening is effectively unobstructed and has a lip projecting from the second wall aspect at the distal end beyond the opening and curving inwardly toward the first wall aspect.

The present invention in another aspect provides an endotracheal tube comprising a tubular member extending between a proximal end and a distal end and having a single opening across the distal end, the tubular member defining an airway lumen therethrough for flow of gas between the proximal end and the opening at the distal end, the tubular member having a generally pre-defined curvature so as to define a first wall aspect and a second, oppositely disposed wall aspect of the distal end, characterised by the distal end having an incomplete bevel extending across the opening from the first wall aspect toward, but not completely through, the second wall aspect of the tubular member, and a lip projecting from the distal end beyond the opening and curving inwardly toward the first wall aspect.

The invention also provides corresponding methods.

The present invention provides an improved endotracheal tube for orotracheal and nasotracheal intubation, which minimizes the tendency of such tubes to snag, drag, and traumatize anatomical features within the nose, larynx, and/or trachea. The improved endotracheal tube is especially useful in facilitating intubation with blind intubation guides, fiberoptic bronchoscopes, and other orotracheal tube introducers. To this end, and in accordance with the principles of the present invention, the distal tip of the endotracheal tube is provided with only a partial or incomplete posterior or rear-facing bevel which allows the tube to slide down the midline of the rear wall of the larynx without snagging the arytenoid cartilages, which are lateral thereto. The partial posterior bevel leaves a depending projection or lip of the anterior tube wall rather than a chisel point. The partial posterior bevel does not cut completely through the anterior wall of the tube, but instead stops short thereof such as at the inner surface of the anterior wall. The partial bevel may advantageously extend partially into the inner surface of the anterior wall such that the lip has a wall thickness that is thinner than the adjacent anterior wall of the tube. The depending lip thus created is advantageously tapered to facilitate its insertion through a narrow nasal passageway and/or into the narrow opening (glottis) between the vocal cords. The lip is also curved posteriorly back toward the axis of the tube lumen to define a convex bearing surface which will slide easily along a nasal passageway as well as down the inner surface of the epiglottis and the inner surface of the anterior tracheal wall without becoming impacted on the epiglottis, anterior commissure, or tracheal rings. The downwardly extending convex bearing surface of the lip may be aligned with the outer anterior tube wall or offset therefrom, and may include a fin on its inferior aspect to facilitate insertion into the glottis.

The lip is semi-flexible and confined to a short length which curves to about, but generally not further than, the midline axis of the tube's distal opening so that if the lip impacts in the body and flexes upward, it will not occlude the lumen of the tube or obstruct the flow of air to the patient's lungs. In this regard, the thinner wall of the lip relative to the wall of the remainder of the tube enhances the lip's vertical flexibility. The lip will not block the passage of a suction catheter or obstruct the view of a bronchoscope fiberbundle passed through the tube to its tip or beyond. Instead, the lip itself will be flexed backward by such tubular instruments when they contact the lip, and will return to its original shape when they are withdrawn from contact with the lip. To facilitate snag-free, friction-free passage of the tube into and through the nose and/or the glottis, the lip is tapered to a narrowly rounded distal edge and a biocompatible lubricious coating may advantageously be covalently bonded to the forward 25% of the tube, including the inflation cuff. The surface lubricity of the tube thus achieved is uniform and is not readily wiped off through inadvertence or otherwise, such as by sliding the tube through a narrow nostril. Where the lip is simply curved inwardly toward the tube lumen, the angle of the curved lip may be about 25° to 35° measured from a line tangent to the lip relative to the midline axis, and is advantageously at an angle of about 30°. Where the lip includes a portion offset outwardly of the tube and then curves back toward the midline axis, the lip curves at an angle of about 30° to 40°. If additional ventilating capacity is desired, one or two holes, known as "Murphy eyes", may be provided, for example, through opposed sides of the tube wall adjacent the distal tip above and lateral to the posterior bevel.

By virtue of the foregoing, there is thus provided an endotracheal tube which can be passed through the nose or mouth into the larynx and trachea with minimum trauma, minimum friction, and without becoming impacted or snagged on anatomical features of and within the nose, larynx, or trachea, such as the cartilages in the nose and rear wall of the larynx, the vocal cords, vocal folds, epiglottis, and anterior tracheal rings. The same endotracheal tube thus provided can be used with a conventional blade laryngoscope and can also cooperate with a blind intubation guide and with a fiberoptic bronchoscope inserted through the tube to the distal tip thereof or beyond to achieve an unobstructed view of the anatomy directly in front of the tube and to achieve fiberoptic (visually directed) intubation. These and other objects and advantages of the present invention shall be made apparent from the accompanying drawings and the description thereof.

### Brief Description of the Drawings

The accompanying drawings, which are incorporated in and constitute a part of this specification, illustrate embodiments of the invention and, together with the general description of the invention given above and the detailed description of the embodiments given below, serve to explain the principles of the present invention.
Fig. 1 is a left side elevational, partially broken away, view of a first embodiment of an endotracheal tube in accordance with the principles of the present invention;
Fig. 2 is a close-up, left side elevational view of the distal end of the tube of Fig. 1;
Fig. 2A is a view like Fig. 2 showing an alternative bevel and lip;
Fig. 3 is a close-up, front elevational view of the distal end of the tube of Fig. 1;
Figs. 4A and 4B are schematic illustrations, partially cut-away, showing the tube of Fig. 1 in use with a blind intubation guide for purposes of explaining the principles of the present invention;
Figs. 5A, 5B, and 5C are schematic illustrations, partially cut-away, showing the tube of Fig. 1 in use with an orotracheal introducer for purposes of explaining the principles of the present invention;
Fig. 6 is close-up, left side elevation view of the distal end of an alternative embodiment of an endotracheal tube in accordance with the principles of the present invention;
Fig. 7 is a close-up, left side elevational view of the distal end of the tube of Fig. 1 with an offset lip portion;
Fig. 8 is a schematic illustration, partially cut-away, showing the tube of Fig. 1 in use with a fiberoptic bronchoscope for purposes of explaining the principles of the present invention; and
Fig. 9 is a schematic illustration, partially cut-away showing the tube of Fig. 1 in use for nasotracheal intubation.

### Detailed Description of the Drawings

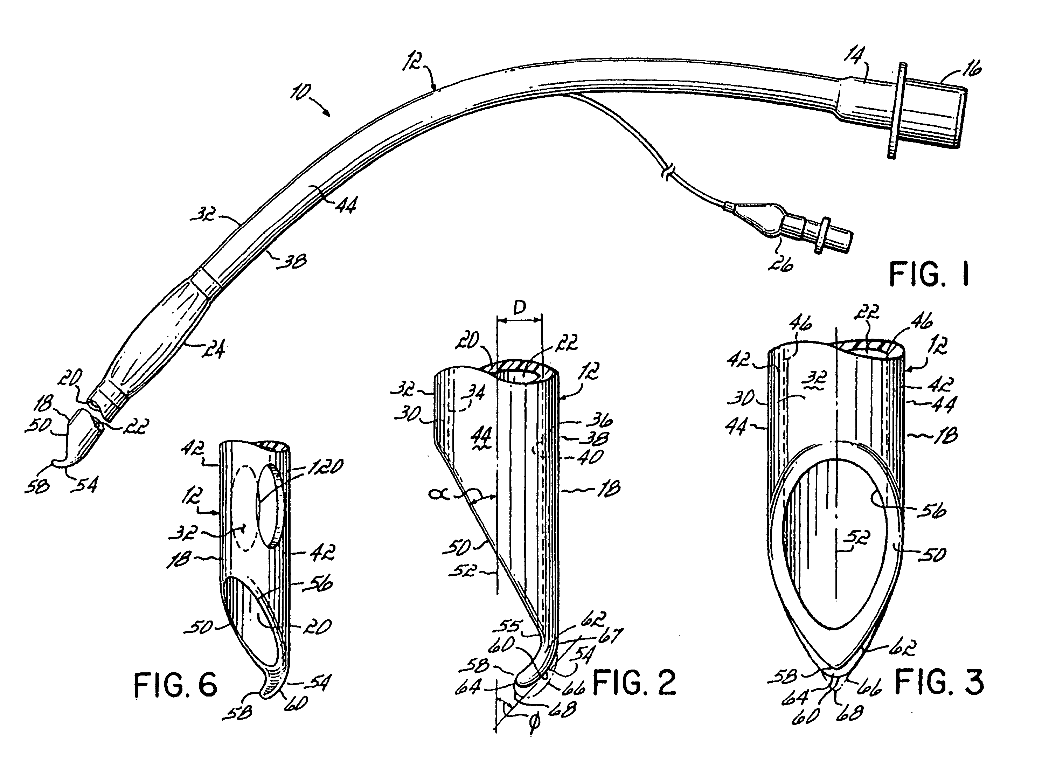

With reference to Figs. 1-3, there is shown an endotracheal tube 10 constructed in accordance with the principles of the present invention. Tube 10 is defined by an elongated plastic tubular member 12 (such as of polyvinyl chloride or plasticized polyvinyl chloride) having a proximal end 14 to which is removably attached a connector piece 16, and a distal tip end 18. The wall 20 of member 12 defines therein a gas flow lumen 22 opening into the proximal end 14 and distal end 18 for flow of gas, such as oxygen, to ventilate a patient. An inflatable cuff or balloon 24 is provided adjacent distal end 18 and coupled to an inflation port 26 as is typical for standard, adult endotracheal tubes. For smaller size tubes, cuff 24 and port 26 may be eliminated.

Tubular member 12 has a generally arcuate shape as seen in Fig. 1. With specific reference to distal tip 18, tube 12 has a posterior wall aspect 30 with outer surface 32 and inner surface 34, an anterior wall aspect 36 with outer surface 38 and inner surface 40, and lateral wall aspects 42 each with an outer surface 44 and an inner surface 46 extending between posterior and anterior wall aspects 30 and 36. The inner surfaces 34, 40, and 46 cooperate to define gas flow lumen 22.

In accordance with the principles of the present invention, tip end 18 is provided with a partial or incomplete posterior (i.e., rear-facing) bevel 50 extending at an angle ∝ of about 37° (measured relative to the midline axis 52 of distal tube end 18) from posterior outer surface 32 toward anterior wall aspect 36, but not completely through wall aspect 36 so as to leave a depending projection or lip 54 of anterior tube wall material 36. Bevel 50 may terminate at inner anterior wall surface 40 as at 55 as seen in Fig. 2 such that the wall thickness of lip 54 is about equal to the thickness of anterior wall aspect 36. To enhance flexibility of lip 54, as shown in Fig. 2A, bevel 50 may continue past surface 40 such that wall thickness of resulting lip 54 is thinner (i.e., between inner surface 104 and bearing surface 60) than anterior wall 36 (i.e., between inner surface 40 and outer surface 38). Where the thinner lip 54 is utilized, lip 54 is advantageously 20% to 25% thinner than anterior wall 36.

Posterior bevel 50 defines an elliptically-shaped, single distal opening 56 (Fig. 3), the major diameter of which intersects midline axis 52 and lip 54. Lip 54 is sized such that its extent from end 55 of bevel 50 to lip free edge 58 is not greater than the distance D between the axis 52 and the inner anterior wall surface 40 so as not to extend past midline axis 52 and not to occlude opening 56 should lip 54 be flexed upward toward that opening. A curvature is advantageously imparted to lip 54 such that lip 54 curves back along a line intersecting the major diameter of opening 56 toward axis 52. Lip 54 projects at an angle ø measured between a line tangent to the midpoint of inferior or outer surface 60 of lip 54 and midline axis 52 of between about 25° and 35°, and advantageously about 30°. Lip 54 has lateral side edges 62 that taper and converge smoothly into free edge 58 which is provided with a rounded bottom 64. A fin 66 (shown in dotted line in Figs. 2 and 3) may be included on outer surface 60 running along the midline axis of lip 54. Fin 66 constitutes a tapered extension of surface 60 and progressively narrows from its origin 67 (confronting bevel end 55) to its free end 68 adjacent lip free edge 58. Lip 54 is semi-flexible so as to yield and flex on impact. By way of example, lip 54 may be flexed backward away from bevel 50 by instruments (not shown) passed through tube 10 and beyond bevel 50, but will return to its original position when such instruments are withdrawn from contact with lip 54. Thus, it may be seen that opening 56 is, effectively, unobstructed.

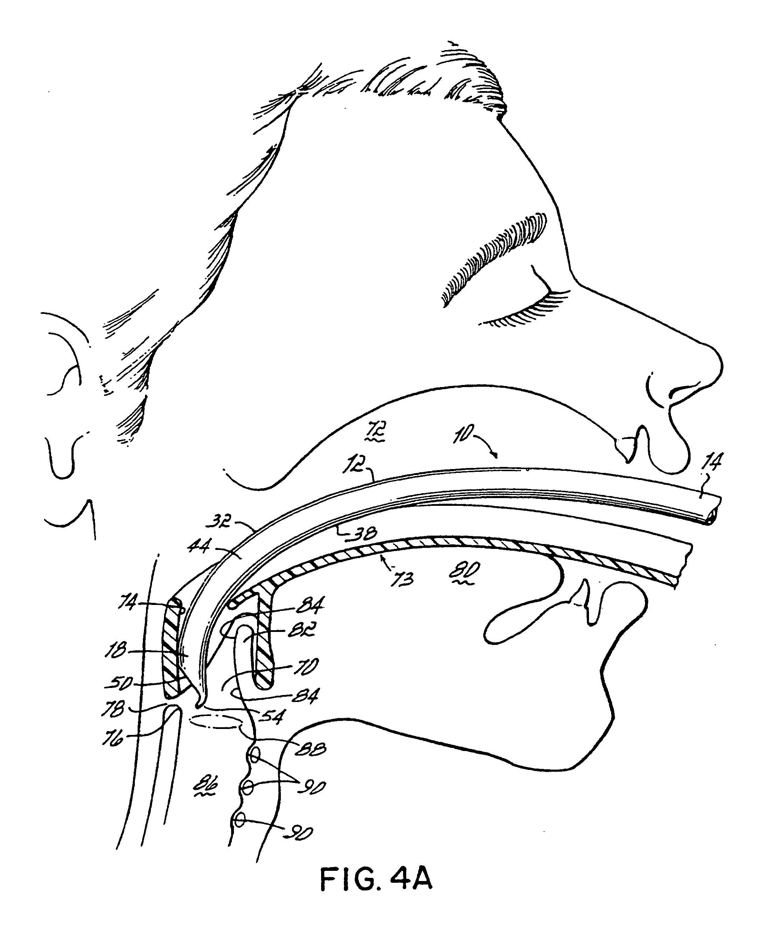

Lip 54 and cuff 24 and the portion of tube 12 therebetween, constituting about the forward 25% of tube 10, are advantageously provided with a biocompatible lubricious coating covalently bonded to their surfaces, such as that supplied by SurModics, Inc. of Eden Prairie, Minnesota based on technology disclosed in U.S. Patent No. 5,637,460 and related patents. The lubricious coating reduces the friction between tube 12 and anatomical structures through which it passes, such as vocal cords 88 and trachea 86 (Fig. 4A); between tube 12 and devices through which it passes, such as blind intubation guide 73 (Fig. 4A); and between tube 12 and medical instruments which pass through it, such as introducer 100 (Fig. 5A). Immediately prior to use of tube 10, the lubricious coating is activated by wetting it with a dip or spray of water or saline. An alternative form of tube lubrication immediately prior to use thereof is to manually apply a film of water-soluble, biocompatible lubricant, such as SURGILUBE, available from Altana, Inc. in Melville, New York.

The incomplete bevel 50 and lip 54 of endotracheal tube 10 make it particularly suitable for those situations in which the tube is to enter the laryngeal opening 70 of a human or animal throat 72 from above as will now be described with reference to Figs. 4A-5C. To this end, when intubation of a patient is desired to be accomplished with a blind intubation guide 73 (such as that shown in the aforementioned '805 patent and/or '254 patent), a guide wall 74 (shown schematically in Figs. 4A and 4B) is caused to be positioned relative to the posterior edge 76 of the laryngeal opening 70 so as to be, in effect, contiguous therewith (i.e., such that any gap 78 thereat is not sizable enough for the tip end 18 of tube 10 to pass therethrough). The guide wall 74 thus defines a bearing surface along which an endotracheal tube may be directed into the laryngeal opening 70. To this end, the posterior bevel 50 may be seen as facing, and thus sliding along, guide wall 74 as tube 10 passes towards laryngeal opening 70 as shown schematically in Fig. 4A (note that cuff 24 has been eliminated for sake of simplicity). As may also be envisioned from Fig. 4A, the curvature of lip 54 causes outer surface 60 and/or fin 66 thereof to serve as a bearing surface to slide along aspects of the guide device that pass over the tongue 80 and epiglottis 82.

The central position of lip 54 further serves to facilitate the trajectory of tip end 18 into and through laryngeal opening 70. In this regard, and as will be appreciated from Figs. 4A and 4B, while posterior bevel 50 helps to slide tube tip 18 over the posterior edge 76 of opening 70, curved lip 54 and/or fin 66 serve to avoid snagging of tube tip 18 on the rear surface 84 of epiglottis 82, the vocal cords (as represented by glottis 88), and/or the cartilaginous rings 90 within trachea 86 (only three shown). Thus, the curvature of outer surface 60 of lip 54 and/or fin 66 provides a bearing surface that deflects the tube tip 18 in a sliding manner off from and/or along those anatomical processes such that tube tip 18 may be fully inserted into trachea 86 without snagging along the way. More specifically, the curvature of lip 54 helps orient the free edge 58 such that it is aligned with the midline of the posterior commissure of the glottis 88 (where the space between the vocal cords is greatest), and is aimed away from the anterior commissure of the glottis 88 (where the space between the vocal cords is narrowest).

If tube tip 18 does become snagged on any structure, such as laryngeal cartilages 76, the thinness and curvature of lip 54 predispose lip 54 to flex vertically (toward posterior tube wall 30) as tube 120 is advanced. Such flexion causes free edge 58 to be deflected inward toward the tube lumen 22 and away from engagement with the anatomical site where it was snagged. As free edge 58 curves inward, curved bearing surface 60 is rotated into contact with the anatomy immediately adjacent the snag site and, assisted by the lubricious coating on surface 60 and forward pressure on tube 10, surface 60 tends to slide away from the snag site toward an area of less resistance at which tube tip 18 is generally directed, such as glottic opening 88. The fin 66 is useful to help wedge apart a narrow glottic opening.

Tube 10 is pushed against guide wall 74 such that tube tip 18 passes into and beyond laryngeal opening 70. The tube continues to be pushed therealong until seated within trachea 86 as desired. Thereafter, guide wall 74 may be removed from throat 72 while leaving tube 10 in place.

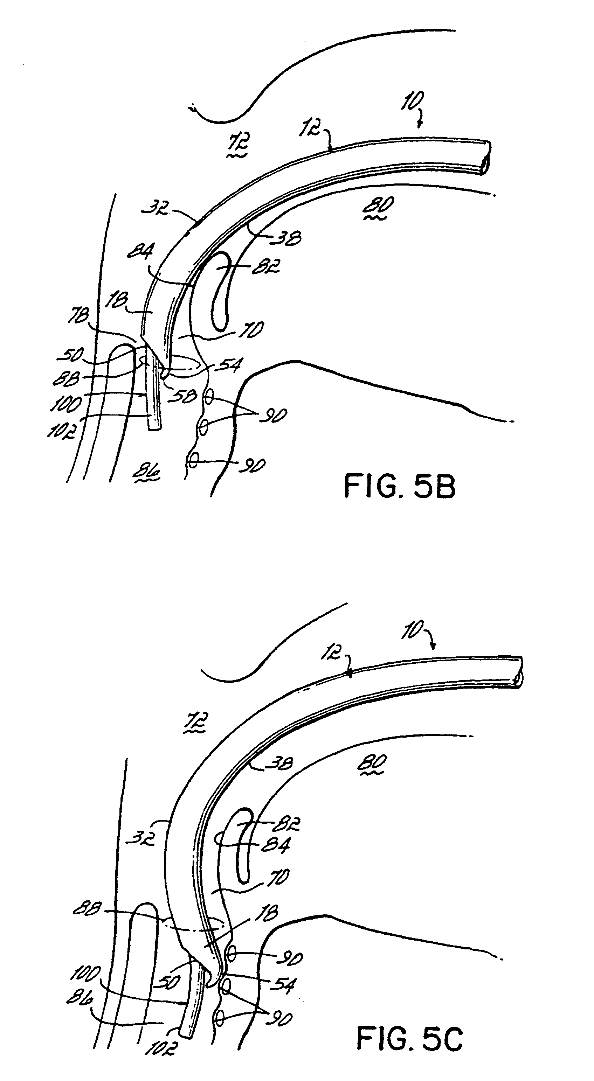

The partial bevel 50 and lip 54 of tube 10 also facilitate intubation with an orotracheal introducer (such as a tubular fiberbundle 100 of a laryngoscope) as will now be described with reference to Figs. 5A, 5B, and 5C. To this end, introducer 100 is inserted into tube 10 from proximal end 14 thereof such that a distal portion 102 of introducer 100 projects out beyond distal tube end 18. Distal end 102 of introducer 100 is inserted into the trachea 86 and tube 10 is then railroaded over introducer 100. The curvature of lip 54 causes the free edge 58 thereof to be turned away from epiglottis 82 as tube tip 18 passes thereover to avoid snagging on epiglottis 82 and to facilitate sliding of bearing surface 60 and tube surface 38 down the rear surface 84 of epiglottis 82. As tube 10 is railroaded over introducer 100, bevel 50 will pass against posterior edge 78 of laryngeal opening 70 at which time rear-facing bevel 50 helps tube tip 18 pass thereover and into laryngeal opening 70 without snagging on edge 76. The curved lip 54, meanwhile, provides a bearing surface 60 to help slide between the vocal cords at glottis 88 without snagging, as exemplified schematically in Fig. 5B (in which event, fin 66 is not necessary). Tube 10 is seated down in the trachea 86 by further railroading along introducer 100 such that bearing surface 60 of curved lip 54 will slide along cartilaginous rings 90 as tube tip 18 passes into the trachea (Fig. 5C). Thereafter, introducer 100 may be removed by pulling it out from the proximal end 14 of tube 12.

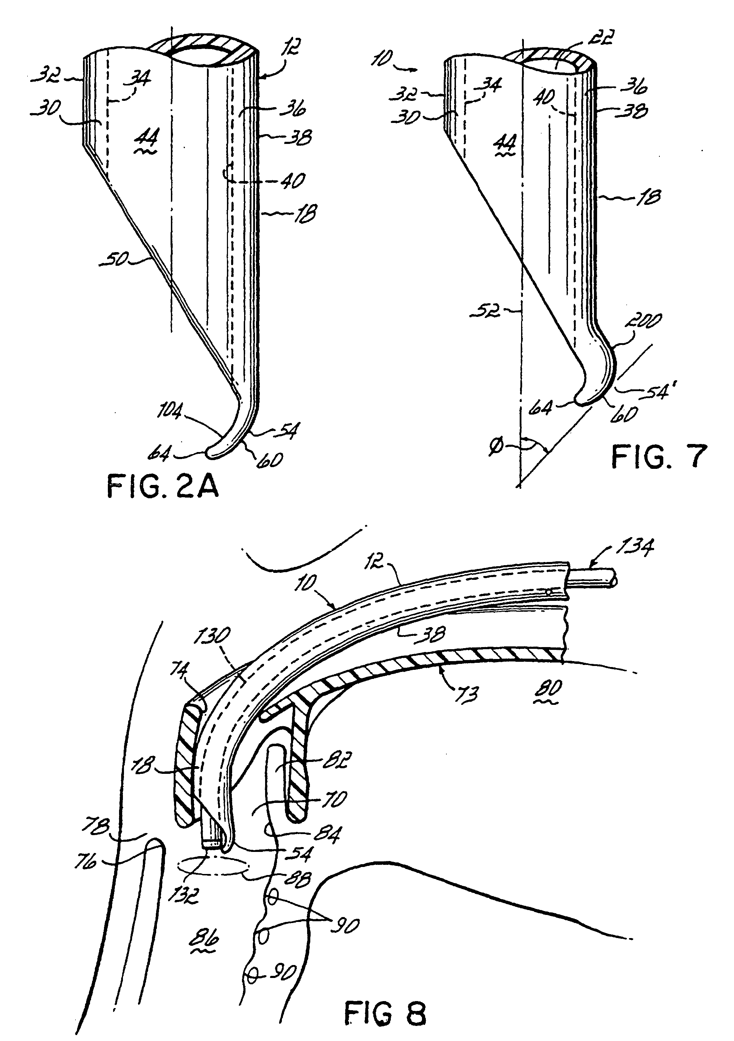

Tube 10 can also cooperate with intubation guide 73 and bronchoscope 134 to achieve inspection of larynx 70 and/or visually directed intubation of trachea 86 as will now be described with reference to Fig. 8. Tube 10 is wetted with water or saline if it is desired to activate the lubricious coating on the surfaces of tube 10. Fiberbundle 130 of bronchoscope 134 is inserted into proximal end 14 of tube 10 and advanced until objective lens 132 of fiberbundle 130 is situated adjacent lip 54 of tube tip 18. Tube 10 is then situated in intubation guide 73 which is seated adjacent larynx 70. Gentle traction may be applied to guide 73 if necessary to align lens 132 with glottis 88, thereby allowing an image of glottis 88 to be transmitted through lens 132 and fiberbundle 130 to bronchoscope 134. While watching this image, tube 10 and fiberbundle 130 may then, optionally, be advanced together through guide 73 and glottis 88 into trachea 86 to achieve tracheal intubation. Thereafter, fiberbundle 130 may be removed from tube 10 by pulling it out from proximal end 14 of tube 10.

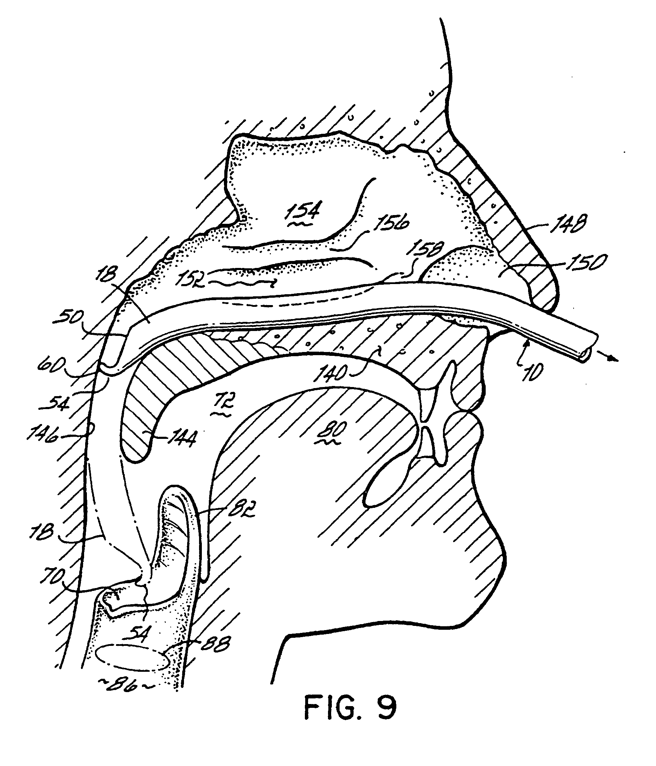

Tube 10 is also useful for blind nasotracheal intubation, as will now be described with reference to Fig. 9. As above, tube 10 is wetted to activate the lubricious coating. Distal end 18 of tube 10 is inserted into nostril 150 of nose 148 and advanced toward posterior pharyngeal wall 146, preferably along inferior meatus 158, between inferior concha 152 and hard palate 140. An alternate pathway could be along middle meatus 156, between inferior concha 152 and middle concha 154. Lip 54 of tube tip 18 passes over soft palate 142 and contacts wall 146. Pressure applied to advance tube 10 causes lip 54 to flex upward against wall 146 so that curved bearing surface 60 comes into contact with wall 146 and slides downward along wall 146, assisted by the lubricious coating on surface 60. As tube 10 is further advanced, past uvula 144 and down along wall 146, tip end 18 rotates into position parallel to wall 146, removing lip 54 from contact with wall 146 and allowing lip 54 to spring back to its original shape (as shown in phantom in Fig. 9). As curved tube 10 slides tangent to wall 146, distal end 18 is directed away from wall 146 and sequentially into larynx 70, glottis 88, and trachea 86. Guidance of tube 10 toward this objective is facilitated by the operator (not shown) listening at the proximal end 14 of tube 10 for breath sounds transmitted from distal end 18, so that the operator will know if tube end 18 is approaching larynx 70 and trachea 86 and should be advanced, or is deviating therefrom and should be slightly withdrawn and redirected.

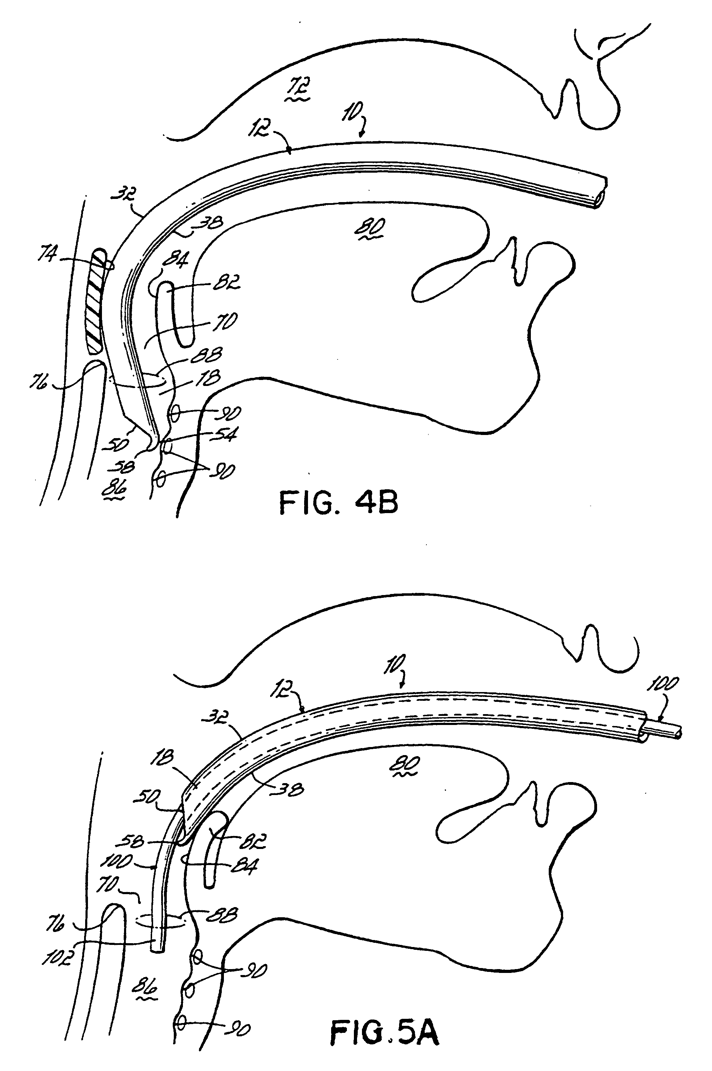

In some applications, it may be helpful to provide additional ventilating capacity through distal end 18, in addition to opening 56. To this end, and with reference to Fig. 6, distal end 18 may be modified by inclusion of a hole or Murphy eye 120 formed completely through a lateral wall aspect 42 so as to be situated just above and lateral to posterior bevel 50, and below cuff 24. A pair of Murphy eyes may be positioned in confronting relationship such that the lateral disposition thereof provides alternate pathways for gas flow to the right and left mainstream bronchi (not shown) in case the opening 56 becomes occluded.

With reference to Fig. 7, a further alternative embodiment is shown of the distal end 18 of tube 10 in which lip 54' thereof is modified from lip 54 of the prior embodiments in that lip 54' includes a portion 200 that is offset outwardly of midline axis 52 and protrudes beyond the cylinder of outer surface 38 of anterior wall aspect 36. Lip 54' then curves back toward midline axis 52 and posterior wall aspect 30. Due to offset portion 200, however, the extent of curvature of lip 54' is such that tip 64 thereof is substantially aligned with inner surface 40 of wall aspect 36 and so does not protrude into the pathway of lumen 22. Lip 54' projects at an angle ø measured between a line tangent to the midpoint of inferior or outer surface 60 of lip 54' and midline axis 52 of between about 30° and 40°.

In use, an endotracheal tube 10 of appropriate size for the recipient individual is selected and the lubricious coating is activated or applied. Lubricated tube 10 (with or without Murphy eyes 120 and/or fin 66 as desired) is inserted into the trachea 86 by use of a blind intubation guide device having a guide wall 74 as described in the aforesaid '805 patent and/or '254 patent, after which the guide device is removed. Alternatively, lubricated tube 10 is inserted into nostril 150 and advanced against meatus 158 and downward along wall 146 into larynx 70 and trachea 86. Further alternatively, an introducer 100 or bronchoscope fiberbundle 130 is inserted through lubricated tube 10 to its tip or beyond and positioned above larynx 70 or into trachea 86, as desired. Tube 10 is then advanced with or railroaded downward thereover and into the trachea 86. Introducer 100 or fiberbundle 130 may then be withdrawn through tube proximal end 14. In all of these cases, the incomplete rear-facing bevel 50 and the depending lip 54 allow the tube 10 to be readily inserted into and advanced in the nose 148, throat 72 and trachea 86 without snagging on or frictionally traumatizing anatomical features of and within the nose, larynx, and trachea.

By virtue of the foregoing, there is thus provided an endotracheal tube that facilitates blind nasotracheal intubation and blind or visual orotracheal intubation without snagging on or frictionally traumatizing anatomical features of and within the nose, larynx, and trachea.

While the present invention has been illustrated by the description of embodiments thereof, and while the embodiments have been described in considerable detail, it is not intended to restrict or in any way limit the scope of the appended claims to such detail. Additional advantages and modifications will readily appear to those skilled in the art. For example, lip 54 or 54' may be provided without a bevel 50 to tube end 18. Alternatively, lip 54 or 54' may not include fin 66. Further, a barium sulfate strip (not shown) may be included in wall 20 for x-ray visualization. The invention in its broader aspects is, therefore, not limited to the specific details, representative apparatus and method, and illustrative examples shown and described.

## Claims

1. An endotracheal tube (10) comprising a tubular member (12) extending between a proximal end (14) and a distal end (18) and having a single opening (56) across the distal end (18), the tubular member (12) having a generally predefined curvature with a first wall aspect (30) and a second. oppositely disposed wall aspect (36) along its length defining an airway lumen (22) therebetween for flow of gas between the proximal end (14) and the opening (56), **characterised in that** the opening (56) is effectively unobstructed and has a lip (54) projecting from the second wall aspect (36) at the distal end (18) beyond the opening (56) and curving inwardly toward the first wall aspect (30).

2. An endotracheal tube (10) comprising a tubular member (12) extending between a proximal end (14) and a distal end (18) and having a single opening (56) across the distal end (18), the tubular member (12) defining an airway lumen (22) therethrough for flow of gas between the proximal end (14) and the opening (56) at the distal end (18), the tubular member (12) having a generally pre-defined curvature so as to define a first wall aspect (30) and a second, oppositely disposed wall aspect (36) of the distal end (18), **characterised by** the distal end (18) having an incomplete bevel (50) extending across the opening (56) from the first wall aspect (30) toward, but not completely through, the second wall aspect (36) of the tubular member (12), and a lip (54) projecting from the distal end (18) beyond the opening (56) and curving inwardly toward the first wall aspect (30).

3. An endotracheal tube as claimed in any preceding Claim wherein the distal end (18) has a longitudinal midline axis (52), the lip (54) curving inwardly toward the midline axis (52).

4. An endotracheal tube as claimed in any preceding Claim wherein the lip (54) tapers to a free edge (58).

5. An endotracheal tube as claimed in any preceding Claim, the distal end (18) having a longitudinal midline axis (52) spaced a distance D from an inner surface (40) of the tubular member (12), the lip (54) extending to a free edge (58) a distance not more than D whereby not to occlude the opening (56) at the distal end (18).

6. An endotracheal tube as claimed in any preceding Claim wherein the lip (54) has an outer surface (60), the tube further comprising a fin (66) extending along the lip outer surface (60).

7. An endotracheal tube as claimed in any preceding Claim, the lip (54) including a portion (200) being offset outwardly of an outside surface (38) of the tubular member (12).

8. An endotracheal tube as claimed in any preceding Claim further comprising a Murphy eye (120) extending through the tubular member (12) and positioned just above and lateral to the opening (56).

9. An endotracheal tube as claimed in any preceding Claim the lip (54) being thinner than an anterior wall (36) of the tubular member (12) at the distal end (18).

10. An endotracheal tube as claimed in any preceding Claim further comprising a lubricious coating covalently bonded to at least the lip (54).

11. An endotracheal tube as claimed in any preceding Claim further comprising an inflation cuff (24) associated with the tubular member (12).

12. An endotracheal tube as claimed in Claim 11 further comprising a lubricious coating covalently bonded to the inflation cuff (24).

13. An endotracheal tube as claimed in any preceding Claim, the lip (54) being semi-flexible.

14. An endotracheal tube as claimed in any preceding Claim wherein the first wall aspect (30) defines a posterior aspect of the tubular member (12) and the second wall aspect (36) defines an anterior aspect of the tubular member (12).

15. A method of forming an endotracheal tube (10) having a proximal end (14) and a distal end (18) with a single opening (56) thereat, the method comprising providing an effectively unobstructed opening (56) and being **characterised by** giving the tube (10) a generally predefined curvature with a first wall aspect (30) and a second, oppositely disposed wall aspect (36) along its length, defining a lip (54) projecting from the second wall aspect (36) at the distal end (18) beyond the opening (56), and curving the lip (54) inwardly toward the first wall aspect (30).

16. A method of forming an endotracheal tube (10) having a proximal end (14) and a distal end (18) with a single opening (56) thereat, and a generally arcuate shape of the tube defining first (30) and second, opposite (36) wall aspects thereof at the distal end (18), the method comprising forming a bevel (50) across the opening (56) at the distal end (18), **characterised in that** the bevel (50) is incomplete and extends from the first wall aspect (30) to, but not completely through, the second wall aspect (36), by defining a lip (54) projecting from the second wall aspect (36) beyond the incomplete bevel (50), and by curving the lip (54) inwardly toward the first wall aspect (30).

17. A method as claimed either in Claim 15 or Claim 16 wherein the distal end (18) has a longitudinal midline axis (52), the method further comprising curving the lip (54) toward the midline axis (52).

18. A method as claimed in any of Claims 15 through 17 further comprising offsetting a portion (200) of the lip (54) outwardly of an outside surface (38) of the tube (10).

19. A method as claimed in any of Claims 15 through 18 further comprising tapering the lip (54) to a free edge (58).

20. A method as claimed in any of Claims 15 through 19 further comprising defining a fin (66) on the lip (54).

21. A method as claimed in any of Claims 15 through 20 further comprising forming a Murphy eye (120) through the tube (10) just above the opening (56).

22. A method as claimed in any of Claims 15 though 20 further comprising forming the lip (54) thinner than an anterior wall (36) of tubular member (12) at distal end (18).

## Patentansprüche

1. Endotrachealtubus (10), umfassend ein röhrenförmiges Element (12), das zwischen einem proximalen Ende (14) und einem distalen Ende (18) verläuft, mit einer einzelnen Öffnung (56) über dem distalen Ende (18), wobei das röhrenförmige Element (12) eine im Allgemeinen vordefinierte Krümmung hat, wobei ein erster Wandaspekt (30) und ein zweiter, gegenüberliegender Wandaspekt (36) über seine Länge ein Luftweglumen (22) dazwischen für einen Gasfluss zwischen dem proximalen Ende (14) und der Öffnung (56) definieren, **dadurch gekennzeichnet, dass** die Öffnung (56) effektiv unblockiert ist und eine Lippe (54) hat, die vom zweiten Wandaspekt (36) am distalen Ende (18) über die Öffnung (56) hinaus vorspringt und einwärts in Richtung auf den ersten Wandaspekt (30) gekrümmt ist.

2. Endotrachealtubus (10), umfassend ein röhrenförmiges Element (12), das zwischen einem proximalen Ende (14) und einem distalen Ende (18) verläuft, mit einer einzelnen Öffnung (56) über dem distalen Ende (18), wobei das röhrenförmige Element (12) ein durch sich verlaufendes Luftweglumen (22) für einen Gasfluss zwischen dem proximalen Ende (14) und der Öffnung am distalen Ende (18) definiert, wobei das röhrenförmige Element (12) eine im Allgemeinen vordefinierte Krümmung hat, um einen ersten Wandaspekt (30) und einen zweiten, gegenüberliegenden Wandaspekt (36) am distalen Ende (18) zu definieren, **dadurch gekennzeichnet, dass** das distale Ende (18) eine unvollständige Abschrägung (50) hat, die über die Öffnung (56) von dem ersten Wandaspekt (30) in Richtung auf, aber nicht komplett durch den zweiten Wandaspekt (36) des röhrenförmigen Elements (12) verläuft, sowie eine Lippe (54), die vom distalen Ende (18) über die Öffnung (56) hinaus verläuft und nach innen in Richtung auf den ersten Wandaspekt (30) gekrümmt ist.

3. Endotrachealtubus nach einem der vorherigen Ansprüche, wobei das distale Ende (18) eine Längsmittellinienachse (52) hat, wobei sich die Lippe (54) nach innen in Richtung auf die Mittellinienachse (52) krümmt.

4. Endotrachealtubus nach einem der vorherigen Ansprüche, wobei sich die Lippe (54) zu einem freien Rand (58) hin verjüngt.

5. Endotrachealtubus nach einem der vorherigen Ansprüche, wobei das distale Ende (18) eine Längsmittellinienachse (52) hat, die über eine Strecke D von einer Innenfläche (40) des röhrenförmigen Elements (12) beabstandet ist, wobei die Lippe (54) zu einem freien Rand (58) hin über eine Strecke verläuft, die nicht größer als D ist, so dass die Öffnung (56) am distalen Ende (18) nicht versperrt wird.

6. Endotrachealtubus nach einem der vorherigen Ansprüche, wobei die Lippe (54) eine Außenfläche (60) hat, wobei der Tubus ferner eine Rippe (66) umfasst, die über die Außenfläche (60) der Lippe verläuft.

7. Endotrachealtubus nach einem der vorherigen Ansprüche, wobei die Lippe (54) einen Abschnitt (200) beinhaltet, der von einer Außenseite (38) des röhrenförmigen Elementes (12) nach außen hin versetzt angeordnet ist.

8. Endotrachealtubus nach einem der vorherigen Ansprüche, ferner umfassend ein Murphy-Auge (120), das durch das röhrenförmige Element (12) verläuft und gerade über der und seitlich zur Öffnung (56) positioniert ist.

9. Endotrachealtubus nach einem der vorherigen Ansprüche, wobei die Lippe (54) dünner ist als eine Vorderwand (36) des röhrenförmigen Elements (12) am distalen Ende (18).

10. Endotrachealtubus nach einem der vorherigen Ansprüche, ferner umfassend eine Gleitbeschichtung, die wenigstens an die Lippe (54) kovalent gebunden ist.

11. Endotrachealtubus nach einem der vorherigen Ansprüche, ferner umfassend eine Aufblähmanschette (24), die mit dem röhrenförmigen Element (12) verbunden ist.

12. Endotrachealtubus nach Anspruch 11, ferner umfassend eine Gleitbeschichtung, die an die Aufblähmanschette (24) kovalent gebunden ist.

13. Endotrachealtubus nach einem der vorherigen Ansprüche, wobei die Lippe (54) halbflexibel ist.

14. Endotrachealtubus nach einem der vorherigen Ansprüche, wobei der erste Wandaspekt (30) einen hinteren Aspekt des röhrenförmigen Elementes (12) definiert und der zweite Wandaspekt (36) einen vorderen Aspekt des röhrenförmigen Elementes (12) definiert.

15. Verfahren zur Herstellung eines Endotrachealtubus (10) mit einem proximalen Ende (14) und einem distalen Ende (18) mit einer einzelnen Öffnung (56) daran, wobei das Verfahren das Bereitstellen einer effektiv unblockierten Öffnung (56) umfasst und **dadurch gekennzeichnet ist, dass** der Tubus (10) eine allgemein vordefinierte Krümmung mit einem ersten Wandaspekt (30) und einem zweiten, gegenüberliegenden Wandaspekt (36) über seine Länge erhält, eine Lippe (54) definiert wird, die vom zweiten Wandaspekt (36) am distalen Ende (18) über die Öffnung (56) hinaus vorsteht, und die Lippe (54) einwärts in Richtung auf den ersten Wandaspekt (30) gekrümmt wird.

16. Verfahren zur Herstellung eines Endotrachealtubus (10) mit einem proximalen Ende (14) und einem distalen Ende (18) mit einer einzelnen Öffnung (56) daran, wobei eine allgemein gebogene Form des Tubus einen ersten (30) und einen zweiten, gegenüberliegenden (36) Wandaspekt davon am distalen Ende (18) definiert, wobei das Verfahren das Bilden einer Abschrägung (50) über die Öffnung (56) am distalen Ende (18) umfasst, **dadurch gekennzeichnet, dass** die Abschrägung (50) unvollständig ist und von dem ersten Wandaspekt (30) zu dem, aber nicht vollständig durch den zweiten Wandaspekt (36) verläuft, eine Lippe (54) definiert wird, die vom zweiten Wandaspekt (36) über die unvollständige Abschrägung (50) hinaus vorsteht, und die Lippe (54) einwärts in Richtung auf den ersten Wandaspekt (30) gekrümmt wird.

17. Verfahren nach Anspruch 15 oder Anspruch 16, wobei das distale Ende (18) eine Längsmittellinienachse (52) hat, wobei das Verfahren ferner das Krümmen der Lippe (54) in Richtung auf die Mittellinienachse (52) umfasst.

18. Verfahren nach einem der Ansprüche 15 bis 17, ferner umfassend das Versetzen eines Abschnitts (200) der Lippe (54) von einer Außenseite (38) des Tubus (10) nach außen hin.

19. Verfahren nach einem der Ansprüche 15 bis 18, ferner umfassend das Verjüngen der Lippe (54) zu einem freien Rand (58) hin.

20. Verfahren nach einem der Ansprüche 15 bis 19, ferner umfassend das Definieren einer Rippe (66) an der Lippe (54).

21. Verfahren nach einem der Ansprüche 15 bis 20, ferner umfassend das Bilden eines Murphy-Auges (120) durch den Tubus (10) gerade über der Öffnung (56).

22. Verfahren nach einem der Ansprüche 15 bis 20, ferner umfassend das Ausbilden der Lippe (54) in einer Weise, dass sie dünner als eine Vorderwand (36) des röhrenförmigen Elementes (12) am distalen Ende (18) ist.

## Revendications

1. Tube endotrachéal (10) comprenant un membre tubulaire (12) s'étendant entre une extrémité proximale (14) et une extrémité distale (18) et ayant une seule ouverture (56) en travers de l'extrémité distale (18), le membre tubulaire (12) ayant une incurvation généralement prédéfinie avec un premier aspect de paroi (30) et un deuxième aspect de paroi disposée de manière opposée (36) le long de sa longueur, définissant une lumière de passage d'air (22) entre celles-ci pour le flux de gaz entre l'extrémité proximale (14) et l'ouverture (56), **caractérisé en ce que** l'ouverture (56) est effectivement dégagée et a une lèvre (54) faisant saillie du deuxième aspect de paroi (36) à l'extrémité distale (18) au-delà de l'ouverture (56) et s'incurvant vers l'intérieur, vers le premier aspect de paroi (30).

2. Tube endotrachéal (10) comprenant un membre tubulaire (12) s'étendant entre une extrémité proximale (14) et une extrémité distale (18) et ayant une seule ouverture (56) en travers de l'extrémité distale (18), le membre tubulaire (12) définissant une lumière de passage d'air (22) à travers celui-ci pour le flux de gaz entre l'extrémité proximale (14) et l'ouverture (56) à l'extrémité distale (18), le membre tubulaire (12) ayant une incurvation généralement prédéfinie de manière à définir un premier aspect de paroi (30) et un deuxième aspect de paroi disposée de manière opposée (36) de l'extrémité distale (18), **caractérisé par** l'extrémité distale (18) ayant un chanfrein incomplet (50) s'étendant en travers de l'ouverture (56) du premier aspect de paroi (30) vers, mais pas complètement à travers le deuxième aspect de paroi (36) du membre tubulaire (12), et une lèvre (54) faisant saillie de l'extrémité distale (18) au-delà de l'ouverture (56) et s'incurvant vers l'intérieur, vers le premier aspect de paroi (30).

3. Tube endotrachéal tel que revendiqué dans l'une quelconque des revendications précédentes, dans lequel l'extrémité distale (18) a un axe central longitudinal (52), la lèvre (54) s'incurvant vers l'intérieur, vers l'axe central (52).

4. Tube endotrachéal tel que revendiqué dans l'une quelconque des revendications précédentes, dans lequel la lèvre (54) diminue vers un bord libre (58).

5. Tube endotrachéal tel que revendiqué dans l'une quelconque des revendications précédentes, l'extrémité distale (18) ayant un axe central longitudinal (52) espacé d'une distance D d'une surface intérieure (40) du membre tubulaire (12), la lèvre (54) s'étendant jusqu'à un bord libre (58) à une distance de pas plus de D, de manière ainsi à ne pas occlure l'ouverture (56) à l'extrémité distale (18).

6. Tube endotrachéal tel que revendiqué dans l'une quelconque des revendications précédentes, dans lequel la lèvre (54) a une surface extérieure (60), le tube comprenant en outre une languette (66) s'étendant le long de la surface extérieure (60) de la lèvre.

7. Tube endotrachéal tel que revendiqué dans l'une quelconque des revendications précédentes, la lèvre (54) comportant une partie (200) étant décalée vers l'extérieur d'une surface externe (38) du membre tubulaire (12).

8. Tube endotrachéal tel que revendiqué dans l'une quelconque des revendications précédentes, comprenant en outre un oeillet de Murphy (120) s'étendant à travers le membre tubulaire (12) et positionné juste au-dessus de, et latéral à l'ouverture (56).

9. Tube endotrachéal tel que revendiqué dans l'une quelconque des revendications précédentes, la lèvre (54) étant plus mince qu'une paroi antérieure (36) du membre tubulaire (12) à l'extrémité distale (18).

10. Tube endotrachéal tel que revendiqué dans l'une quelconque des revendications précédentes, comprenant en outre un revêtement glissant lié de manière covalente à la lèvre (54) au moins.

11. Tube endotrachéal tel que revendiqué dans l'une quelconque des revendications précédentes, comprenant en outre un manchon d'insufflation (24) associé au membre tubulaire (12).

12. Tube endotrachéal tel que revendiqué dans l'une quelconque des revendications précédentes, comprenant en outre un revêtement glissant lié de manière covalente au manchon d'insufflation (24).

13. Tube endotrachéal tel que revendiqué dans l'une quelconque des revendications précédentes, la lèvre (54) étant semi-flexible.

14. Tube endotrachéal tel que revendiqué dans l'une quelconque des revendications précédentes, dans lequel le premier aspect de paroi (30) définit un aspect postérieur du membre tubulaire (12) et le deuxième aspect de paroi (36) définit un aspect antérieur du membre tubulaire (12).

15. Méthode de formation d'un tube endotrachéal (10) ayant une extrémité proximale (14) et une extrémité distale (18) avec une seule ouverture (56) au niveau de celle-ci, la méthode comprenant fournir une ouverture effectivement dégagée (56) et étant **caractérisée** en donnant au tube (10) une incurvation généralement prédéfinie avec un premier aspect de paroi (30) et un deuxième aspect de paroi disposée de manière opposée (36) le long de sa longueur, en définissant une lèvre (54) faisant saillie du deuxième aspect de paroi (36) à l'extrémité distale (18) au-delà de l'ouverture (56), et en incurvant la lèvre (54) vers l'intérieur, vers le premier aspect de paroi (30).

16. Méthode de formation d'un tube endotrachéal (10) ayant une extrémité proximale (14) et une extrémité distale (18) avec une seule ouverture (56) au niveau de celle-ci, et une forme généralement arquée du tube définissant un premier aspect de paroi (30) et un deuxième aspect de paroi opposée (36) de celui-ci à l'extrémité distale (18), la méthode comprenant former un chanfrein (50) en travers de l'ouverture (56) à l'extrémité distale (18), **caractérisée en ce que** le chanfrein (50) est incomplet et s'étend du premier aspect de paroi (30) vers, mais pas complètement à travers, le deuxième aspect de paroi (36), en définissant une lèvre (54) faisant saillie du deuxième aspect de paroi (36) au-delà du chanfrein incomplet (50), et en incurvant la lèvre (54) vers l'intérieur, vers le premier aspect de paroi (30).

17. Méthode telle que revendiquée dans la revendication 15 ou la revendication 16, dans laquelle l'extrémité distale (18) a un axe central longitudinal (52), la méthode comprenant en outre incurver la lèvre (54) vers l'axe central (52).

18. Méthode telle que revendiquée dans l'une quelconque des revendications 15 à 17, comprenant en outre décaler une partie (200) de la lèvre (54) vers l'extérieur d'une surface externe (38) du tube (10).

19. Méthode telle que revendiquée dans l'une quelconque des revendications 15 à 18, comprenant en outre faire diminuer la lèvre (54) vers un bord libre (58).

20. Méthode telle que revendiquée dans l'une quelconque des revendications 15 à 19; comprenant en outre définir une languette (66) sur la lèvre (54).

21. Méthode telle que revendiquée dans l'une quelconque des revendications 15 à 20 comprenant en outre former un oeillet de Murphy (120) à travers le tube (10) juste au-dessus de l'ouverture (56).

22. Méthode telle que revendiquée dans l'une quelconque des revendications 15 à 20, comprenant en outre former la lèvre (54) plus mince qu'une paroi antérieure (36) du membre tubulaire (12) à l'extrémité distale (18).
